(19) Europäisches Patentamt
European Patent Office
Office européen des brevets

(11) Veröffentlichungsnummer: **0 571 845 A2**

(12) # EUROPÄISCHE PATENTANMELDUNG

(21) Anmeldenummer: **93107920.6**

(51) Int. Cl.5: **C07C 263/18**

(22) Anmeldetag: **14.05.93**

(30) Priorität: **27.05.92 DE 4217525**

(43) Veröffentlichungstag der Anmeldung:
**01.12.93 Patentblatt 93/48**

(84) Benannte Vertragsstaaten:
**BE DE ES FR GB IT NL PT**

(71) Anmelder: **BAYER AG**

**D-51368 Leverkusen(DE)**

(72) Erfinder: **Müller, Hanns Peter, Dr.**
**Hollweg 20**
**W-5068 Odenthal(DE)**
Erfinder: **Scholl, Hans Joachim, Dr.**
**Am Feldrain 5**
**W-5000 Köln 80(DE)**
Erfinder: **Kapps, Manfred, Dr.**
**Odenthaler Markweg 56**
**W-5060 Bergisch Gladbach 2(DE)**

(54) **Verfahren zur Standardisierung und Stabilisierung von organischen Polyisocyanaten und ihre Verwendung.**

(57) Die Erfindung betrifft ein neues Verfahren zur Standardisierung und Stabilisierung unter gleichzeitiger Reaktivitätserhöhung von organischen Polyisocyanaten durch Vermischen der Polyisocyanate mit bestimmten mindestens eine Epoxidgruppe aufweisenden Verbindungen, gegebenenfalls anschließender Erwärmung, und Stabilisierung der so erhaltenen Gemische mit besonderen Silylierungsmitteln und/oder Alkylierungsmitteln sowie die Verwendung dieser Polyisocyanate.

EP 0 571 845 A2

Die Erfindung betrifft ein neues Verfahren zur Standardisierung und Stabilisierung unter gleichzeitiger Reaktivitätserhöhung von organischen Polyisocyanaten durch Vermischen der Polyisocyanate mit bestimmten mindestens eine Epoxidgruppe aufweisenden Verbindungen und gegebenenfalls anschließender Erwärmung und Stabilisierung der so erhaltenen Gemische mit besonderen Silylierungsmitteln und/ oder Alkylierungsmitteln sowie die Verwendung dieser Polyisocyanate.

Herstellungsbedingte Verunreinigungen wechselnder Art und Menge in Polyisocyanaten sind Anlaß für Aktivitätsschwankungen, deren Auswirkungen bis in entsprechende Folgeprodukte reichen, so daß eine reproduzierbare und damit wirtschaftliche Verwendung erschwert wird. Insbesondere enthalten die bekannten Phosgenierungsprodukte von Anilin-Formaldehyd-Kondensaten (rohe Polyisocyanatgemische der Diphenylmethanreihe) eine Fülle derartiger Verunreinigungen. Nach Chem. Soc. Rev. 3 (1974), S. 209 ff. handelt es sich hierbei vor allem um Chlor enthaltende Verunreinigungen, die immer dann Aktivitätsschwankungen verursachen, wenn es sich um "leicht bewegliches", sogenanntes hydrolysierbares Chlor (HC-Wert) handelt. Eine Verengung der Schwankungsbreite durch Herabsetzung der Menge dieser Verunreinigungen mit der Folge einer Aktivitäts-Normierung und -Verbesserung ist daher sowohl von technischer als auch von wirtschaftlicher Bedeutung.

Gemäß der Lehre der DD 285 593 werden organische Polyisocyanate bei 200 bis 240 °C durch Behandeln mit Acetamidgruppen enthaltenden Verbindungen oder $\epsilon$-Caprolactam und anschließendem Durchblasen von inerten Gasen gereinigt.

Nach der Lehre der GB-PS 10 80 717 gelingt die Herabsetzung des Wertes an hydrolisierbarem Chlor (HC-Wert) durch thermische Behandlung bei 180 bis 220 °C. Unabhängig vom großen Energieaufwand sind derartige Hochtemperaturverfahren wegen der außerordentlichen Reaktivität von Polyisocyanaten, die unter plötzlicher Wärmeabgabe oligomerisieren können, gefährlich.

In der DE-OS 26 14 323 wird ein Verfahren zur Umwandlung von Polyisocyanaten beschrieben, welches als Säureakzeptor ein 1,2-Epoxid verwendet. Dieses Verfahren schreibt aber zwingend die Mitverwendung von Entaktivierungsmitteln wie Halogenwasserstoffen, Halogeniden des Phosphors oder Zinns oder Oxyhalogeniden des Phosphors oder Schwefels vor. Damit enthält die Mischung der resultierenden modifizierten Polyisocyanate jedoch wiederum schwankende Mengen an hydrolisierbarem Chlor.

Die bekannten Verfahren EP 445 608 und EP 445 602 zur Standardisierung von Polyisocyanaten sind unsicher, technisch aufwendig oder erzeugen neue Mängel durch Zusätze.

Aufgabe war es daher, ein technisch einfaches Verfahren zur Reinigung von organischen Polyisocyanaten zur Verfügung zu stellen, welches die angesprochenen Mängel nicht aufweist.

Diese Aufgabe konnte mit dem nachstehend näher beschriebenen erfindungsgemäßen Verfahren gelöst werden.

Das Prinzip des erfindungsgemäßen Verfahrens besteht darin, den zu behandelnden technischen Polyisocyanaten eine geringe Menge von bestimmten mindestens eine Epoxidgruppe aufweisenden Verbindungen zuzusetzen, gegebenenfalls zu erwärmen, und die so erhaltenen Gemische mit bestimmten Silylierungsmitteln und/oder Alkylierungsmitteln zu stabilisieren.

Gegenstand der Erfindung ist ein Verfahren zur Standardisierung und Stabilisierung von organischen Polyisocyanaten, welches dadurch gekennzeichnet ist, daß man organische Polyisocyanate bei 20 °C bis 150 °C mit

a) 0,01 bis 10 Gew.-%, vorzugsweise 0,1 bis 2 Gew.-%, bezogen auf Polyisocyanat, einer mindestens eine Epoxidgruppe aufweisenden organischen Verbindung der Formel (I)

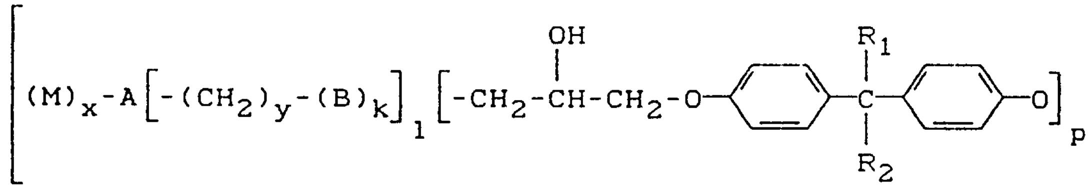

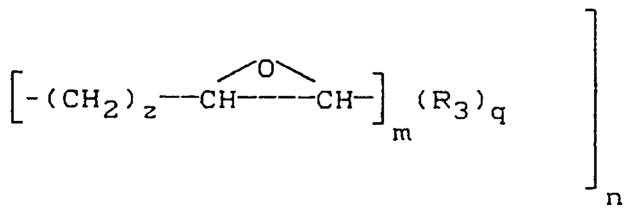

(I)

wobei

x = 0 bis 1
y = 0 bis 4
z = 0 bis 6
k = 0 bis 1
l = 0 bis 8
m = 0,5 bis 4
n = 1 bis 10
p = 0 bis 15
q = 0 bis 1

A = H, ein- oder mehrwertige, gesättigte oder ungesättigte, cyclische oder acyclische Alkyl-, Aryl-, Acyl- oder N-Acylgruppen, gegebenenfalls mit Substituenten,

B = O, S, N

M =

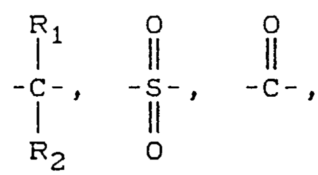

-O-, -S-,

und $R_1$, $R_2$ und $R_3$ für Wasserstoff oder $C_1$- bis $C_4$-Alkylreste stehen,
vermischt und nachfolgend oder gleichzeitig das Umsetzungsprodukt mit
b) 0,01 bis 1 Gew.-%, vorzugsweise 0,01 bis 0,1 Gew.-%, bezogen auf Polyisocyanat, einer silylierten Säure der Formel (II)

$X\text{-}[Si(CH_3)_3]_n$ (II)

und/oder einer alkylierten Säure der Formel (III)

X-[Alkyl]$_n$ (III)

stabilisiert, wobei

X für einen Säurerest steht, wie er durch Entfernen der aciden Wasserstoffatome aus einer n-basischen Sauerstoff enthaltenden Säure mit einem pKa-Wert von max. 2 erhalten wird, und wobei

n für eine ganze Zahl von 1 bis 3 steht.

Besonders bevorzugt werden als organische Polyisocyanate gegebenenfalls chemisch modifizierte Polyisocyanate oder Polyisocyanatgemische der Diphenylmethanreihe eingesetzt.

Als Verbindungen der Formel (II) werden vorzugsweise Trifluormethansulfonsäuretrimethylsilylester oder Phosphorsäuretris(trimethylsilylester) eingesetzt.

Bevorzugt werden als Verbindungen der Formel (III) Benzolsulfonsäuremethylester, Methansulfonsäuremethylester, Trifluormethansulfonsäuremethylester oder Toluolsulfonsäuremethylester eingesetzt.

Die Stabilisatoren (II) und (III) können allein oder gemeinsam in beliebigen Mischungsverhältnissen eingesetzt werden.

Die gemäß dem erfindungsgemäßen Verfahren gereinigten Polyisocyanate werden vorzugsweise als Ausgangsmaterialien zur Kunststoffherstellung verwendet.

Ein weiterer Gegenstand der Erfindung ist die Verwendung der nach dem erfindungsgemäßen Verfahren hergestellten Polyisocyanate als Ausgangsmaterialien bei der Herstellung von Polyurethan-Kunststoffen nach dem Isocyanat-Polyadditionsverfahren, zur Herstellung von modifizierten Polyisocyanaten, zur Herstellung von Polyisocyanurat-Kunststoffen und insbesondere zur Herstellung von Polyurethanschaumstoffen.

Ausgangsmaterialien für das erfindungsgemäße Verfahren sind beliebige organische Polyisocyanate der aus der Polyurethanchemie an sich bekannten Art.

Geeignete Ausgangspolyisocyanate sind beispielsweise (cyclo)aliphatische Diisocyanate wie beispielsweise 1,6-Diisocyanatohexan (HDI), 1-Isocyanato-3,3,5-trimethyl-5-isocyanatomethyl-cyclohexan (IPDI), 4,4'-Diisocyanatodicyclohexylmethan (HMDI) und beliebige Gemische derartiger aliphatische Diisocyanate. Auch aromatische Diisocyanate wie 2,4- und/oder 2,6-Diisocyanatotoluol (TDI) können erfindungsgemäß als Ausgangsmaterial eingesetzt werden. Beliebige modifizierte Polyisocyanate können als Ausgangsmaterialien ebenfalls verwendet werden. Von Interesse sind in diesem Zusammenhang auch Reaktionsgemische, wie sie bei der Trimerisierung eines Teils der Isocyanatgruppen von HDI, IPDI oder Gemischen aus HDI und IPDI zwecks Herstellung der entsprechenden Isocyanuratgruppen aufweisenden Polyisocyanate erhalten werden, und die im wesentlichen aus den genannten Ausgangsdiisocyanaten und den entstehenden Isocyanuratgruppen aufweisenden Polyisocyanaten bestehen.

Besonders bevorzugt kommen jedoch beim erfindungsgemäßen Verfahren gegebenenfalls chemisch modifizierte Polyisocyanate oder Polyisocyanatgemische der Diphenylmethanreihe zum Einsatz. Hierzu gehören beispielsweise die rohen Phosgenierungsprodukte von Anilin/-Formaldehyd-Kondensaten ("Roh-MDI"), aus diesen rohen Gemischen erhaltene Destillationsfraktionen, beispielsweise Polyisocyanate oder Polyisocyanatgemische, die zu 40 bis 100 Gew.-% aus Diisocyanatodiphenylmethan-Isomeren und zu 0 bis 60 Gew.-% aus höher als difunktionellen Polyisocyanaten der Diphenylmethanreihe bestehen, wobei sich die Diisocyanatodiphenylmethan-Isomeren zu 40 bis 100 Gew.-%, vorzugsweise 40 bis 80 Gew.-% aus 4,4'-Diisocyanatodiphenylmethan und mit dem Rest aus 2,4'-Diisocyanatodiphenylmethan und gegebenenfalls 2,2'-Diisocyanatodiphenylmethan zusammensetzen, wobei der Anteil des letztgenannten Diisocyanats bis zu 8 Gew.-%, bezogen auf das Gewicht der Diisocyanate ausmachen kann.

Die genannten Polyisocyanate oder Polyisocyanatgemische können auch in chemisch modifizierter Form zur Anwendung kommen. Unter chemischer Modifizierung sind hierunter insbesondere Urethanisierung, Carbodiimidisierung, Dimerisierung oder Trimerisierung eines Teils der Isocyanatgruppen zu verstehen. Geeignete urethanisierte Ausgangsverbindungen sind insbesondere Umsetzungsprodukte der beispielhaft genannten Polyisocyanate oder Polyisocyanatgemische mit unterschüssigen Mengen an mehrwertigen Alkoholen, insbesondere mit Polypropylenglykolen eines maximalen Molekulargewichts von 700 unter Einhaltung eines NCO/OH-Äquivalentverhältnisses von 10:1 bis 10:3. In Betracht kommende Carbodiimidisierungsprodukte sind insbesondere Carbodiimidgruppen und/oder Uretonimingruppen aufweisende Derivate der beispielhaft genannten Polyisocyanate oder Polyisocyanatgemische, die durch Carbodiimidisierung von 5 bis 30 Gew.-% der Isocyanatgruppen mit Hilfe von Carbodiimidisierungskatalysatoren in an sich bekannter Weise hergestellt worden sind. Geeignete Dimerisierungs- und/oder Trimerisierungsprodukte sind beispielsweise Uretdion- und/oder Isocyanuratgruppen aufweisende Derivate der beispielhaft genannten Polyisocyanate oder Polyisocyanatgemische, die durch Dimerisierung und/oder Trimerisierung unter Uretdion- und/oder Isocyanuratbildung von 10 bis 30 % der Isocyanatgruppen unter Verwendung von bekannten Trimerisierungskatalysatoren hergestellt worden sind. Beliebige Gemische der chemisch modifizierten Polyisocyanate bzw. Polyisocyanatgemische können selbstverständlich ebenfalls eingesetzt werden.

Ganz besonders bevorzugt werden beim erfindungsgemäßen Verfahren rohe Polyisocyanatgemische der Diphenylmethanreihe mit einer Viskosität bei 24°C von 10 bis 800 mPa.s, vorzugsweise 15 bis 400 mPa.s, eingesetzt. Diese besonders bevorzugt als Ausgangsmaterialien eingesetzten Polyisocyanatgemische weisen im allgemeinen einen Gehalt an hydrolysierbarem Chlor (HC-Wert) von 0,01 bis 0,2 Gew.-%, vorzugsweise 0,02 bis 0,1 Gew.-% auf.

Zur Durchführung des erfindungsgemäßen Verfahrens werden die beispielhaft genannten Ausgangspolyisocyanate mit 0,01 bis 10 Gew.-%, vorzugsweise 0,1 bis 2 Gew.-%, der Verbindungen der Formel (I) behandelt, welche mindestens eine Epoxidgruppe aufweisen:

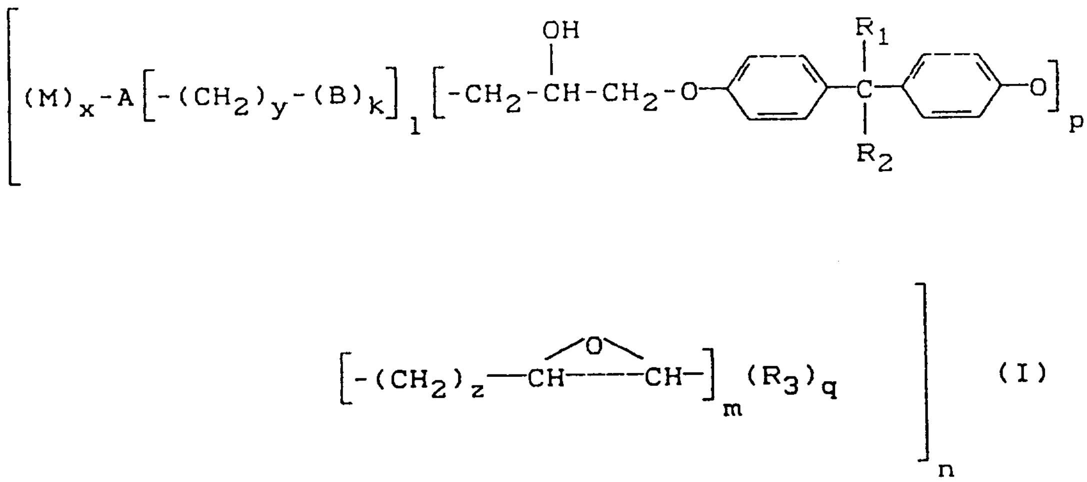

(I)

wobei
x = 0 bis 1
y = 0 bis 4
z = 0 bis 6
k = 0 bis 1
l = 0 bis 8
m = 0,5 bis 4
n = 1 bis 10
p = 0 bis 15
q = 0 bis 1

A = H, ein- oder mehrwertige, gesättigte oder ungesättigte, cyclische oder acyclische Alkyl-, Aryl-, Acyl- oder N-Acylgruppen, gegebenenfalls mit Substituenten,
B = O, S, N
M =

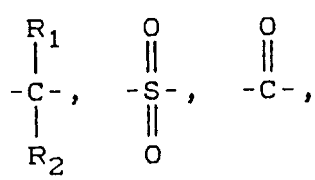

-O-, -S-,

und $R_1$, $R_2$ und $R_3$ für Wasserstoff oder $C_1$- bis $C_4$-Alkylreste stehen.

Derartige Verbindungen sind z.B. Phenoxypropylenoxid, Propylenoxid, Ethylenoxid, Styroloxid, Butylenoxid oder deren Mischungen. Weiterhin kommen beliebige, mindestens zwei Epoxidgruppen enthaltende Verbindungen in Frage, vorzugsweise 1,2-Epoxidgruppen aufweisende aliphatische, cycloaliphatische, aromatische oder heterocyclische Verbindungen. Die als Komponente (I) eingesetzten Polyepoxide weisen bevorzugt pro Molekül 2 bis 4, vorzugsweise 2 Epoxidgruppen und ein Epoxidäquivalentgewicht von 90 bis 500, vorzugsweise 170 bis 220, auf.

Geeignete Polyepoxide sind beispielsweise Polyglycidylether mehrwertiger Phenole, beispielsweise von Brenzkatechin, Resorcin, Hydrochinon, 4,4'-Dihydroxydiphenylmethan, 4,4'-Dihydroxy-3,3'-dimethyldiphenylmethan, 4,4'-Dihydroxydiphenylmethan, 4,4'-Dihydroxydiphenylcyclohexan, 4,4'-Dihydroxy-3,3'-dimethyldiphenylpropan, 4,4'-Dihydroxydiphenyl, 4,4'-Dihydroxydiphenylsulfon, Tris-(4-hydroxyphenyl)-methan, Chlorierungs- und Bromierungsprodukten der vorstehend genannten Diphenole, Novolacken (d.h. aus Umsetzungsprodukten von ein- oder mehrwertigen Phenolen mit Aldehyden, insbesondere Formaldehyd, in

Gegenwart saurer Katalysatoren), Diphenolen, die durch Veresterung von 2 mol des Natriumsalzes einer aromatischen Oxycarbonsäure mit einem mol eines Dihalogenalkans oder Dihalogendialkylethers erhalten wurden (vergl. GB-PS 1 017 612), oder Polyphenolen, die durch Kondensation von Phenolen und langkettigen, mindestens zwei Halogenatome enthaltenen Halogenparaffinen erhalten wurden (vergl. GB-PS 1 024 288). Weiterhin seien genannt: Polyepoxidverbindungen auf der Basis von aromatischen Aminen und Epichlorhydrin, z.B. N-Di-(2,3-epoxypropyl)-anilin, N,N'-Dimethyl-N,N'-diepoxypropyl-4,4'-diaminodiphenylmethyl, N-Diepoxypropyl-4-aminophenylglycid-ether (vergl. GB-PS 772 830 und 816 923).

Außerdem kommen in Frage: Glycidylester mehrwertiger aromatischer: aliphatischer und cycloaliphatischer Carbonsäuren, beispielsweise Phthalsäurediglycidylester, Adipinsäurediglycidylester und Glydidylester von Umsetzungsprodukten aus 1 mol eines aromatischen oder cycloaliphatischen Dicarbonsäureanhydrids und 1/2 mol eines Diols bzw. 1/n mol eines Polyols mit n Hydroxygruppen oder Hexahydrophthalsäurediglycidylester, die gegebenenfalls mit Methylgruppen substituiert sein können.

Glycidylether mehrwertiger Alkohole, beispielsweise von 1,4-Butandiol, 1,4-Butendiol, Glycerin, Trimethylolpropan, Pentaerythrit und Polyethylenglykolen können ebenfalls verwendet werden. Von weiterem Interesse sind Triglycidylisocyanurat, N,N'-Diepoxypropyloxamid, Polyglycidylthioether aus mehrwertigen Thiolen, wie beispielsweise aus Bismercaptomethylbenzol, Diglycidyltrimethylentrisulfon, Polyglycidylether auf Basis von Hydantoinen.

Schließlich seien Epoxidierungsprodukte von mehrfach ungesättigten Verbindungen genannt, wie von vegetabilischen Ölen und deren Umwandlungsprodukten, Epoxidierungsprodukte von Di- und Polyolefinen, wie Butadien, Vinylcyclohexen, 1,5-Cyclooctadien, 1,5,9-Cyclododecatrien, Polymerisaten und Mischpolymerisaten, die noch epoxidierbare Doppelbindungen enthalten, z.B. auf Basis von Polybutadien, Polyisopren, Butadien-Styrol-Mischpolymerisaten, Divinylbenzol, Dicyclopentadien, ungesättigten Polyestern, ferner Epoxidierungsprodukte aus Olefinen, welche durch Diels-Alder-Addition zugänglich sind und anschließend durch Epoxidierung mit Perverbindungen in Polyepoxide überführt werden oder aus Verbindungen, die zwei Cyclopenten- oder Cyclohexenringe über Brückenatome- oder Brückenatomgruppen verknüpft enthalten.

Außerdem seien Polymerisate von ungesättigten Monoepoxiden genannt, beispielsweise aus Methacrylsäureglycidylester oder Allylglycidylether.

Vorzugsweise werden erfindungsgemäß folgende Polyepoxidverbindungen oder deren Mischungen als Komponente (I) verwendet:
Propylenoxid, Phenoxypropylenoxid, Polyglycidylether mehrwertiger Phenole, insbesondere von Bisphenol A: Polyepoxidverbindungen auf der Basis von aromatischen Aminen, insbesondere Bis(N-epoxypropyl)-anilin, N,N'-Dimethyl-N,N'-diepoxypropyl-4,4'-diaminodiphenylmethan und N-Diepoxypropyl-4-amino-phenylglycidylehter; Polyglycidylester aus cycloaliphatischen Dicarbonsäuren, insbesondere Hexahydrophthalsäurediglycidylester und Polyepoxide aus dem Umsetzungsprodukt von n molen Hexahydro-phthalsäureanhydrid und 1 mol eines Polyols mit n Hydroxylgruppen (n = ganze Zahl von 2 bis 6), insbesondere 3 mol Hexahydrophthalsäureanhydrid und einem mol 1,1,1-Trimethylolpropan, 3,4-Epoxycyclohexylmethan-3,4-epoxycyclohexancarboxylat.

Flüssige Polyepoxide oder niedrigviskose Diepoxide, wie Bis-(N-epoxipropyl)-anilin oder Vinylcyclohexandiepoxid können in besonderen Fällen die Viskosität von bereits flüssigen Polyepoxiden weiter herabsetzen oder feste Polyepoxide in flüssige Mischungen überführen.

Bei den Stabilisatoren handelt es sich um Verbindungen der Formel (II)

$X\text{-}[Si(CH_3)_3]_n$ (II)

in der

X für einen Säurerest steht, wie er durch Entfernung der aciden Wasserstoffatome aus einer n-basischen, sauerstoffaufweisenden Säure mit einem pKa-Wert von maximal 2 erhalten wird und

n für eine ganze Zahlt von 1 bis 3 steht. Vorzugsweise geeignet sind beispielsweise entsprechende silylierte Sulfonsäuren wie Trifluormethansulfonsäuretrimethylsilylester, silylierte Ester von Säuren des Phosphors wie Phosphorsäuretris(trimethylsilylester) oder Phosphorsäurediethylestertrimethylsilylester.

Ebenso geeignet als Stabilisatoren sind Verbindungen der Formel (III)

$X\text{-}[Alkyl]_n$ (III)

in der

X für einen Säurerest steht, wie er durch Entfernung der aciden Wasserstoffatome aus einer n-basischen Sauerstoff aufweisenden Säure mit einem pKa-Wert von max. 2 erhalten wird und

n für eine ganze Zahl von 1 bis 3 steht.

Vorzugsweise geeignet sind beispielsweise entsprechende Sulfonsäuren wie Methansulfonsäuremethylester, Trifluormethansulfonsäuremethylester, Toluolsulfonsäuremethylester, Benzolsulfonsäuremethylester.

Das Gemisch aus Polyisocyanat und einer mindestens eine Epoxidgruppe aufweisenden Verbindung wird vorzugsweise 0,5 bis 5 Stunden bei 20 bis 150 °C gehalten, vorzugsweise bei 60 bis 80 °C: anschließend werden zur Stabilisierung der gereinigten Polyisocyanate, Verbindungen der Formel (II) und/oder der Formel (III), gegebenenfalls gelöst im jeweils eingesetzten Polyisocyanat, zugegeben und der Ansatz weitere 0,5 bis 5 Stunden bei 20 bis 150 °C gehalten, vorzugsweise bei 60 bis 100 °C. Es ist ebenso möglich, die Verbindungen der Formel (I), (II) und/oder (III) gleichzeitig dem Ansatz zuzugeben.

Die standardisierten und stabilisierten Polyisocyanate können direkt zur Herstellung von Polyurethankunststoffen nach dem Isocyanatpolyadditionsverfahren verwendet werden. Es ist aber auch möglich, die nach dem erfindungsgemäßen Verfahren durch Zugabe von Verbindungen der Formel (I) gereinigten Polyisocyanate einer Modifizierungsreaktion zu unterwerfen, z.B. durch Urethanisierung, Trimerisierung, Biuretbildung, Dimerisierung oder Allophanantisierung und/oder Carbodimidisierung und anschließend die Stabilisierung mit den Verbindungen der Formel (II) und/oder (III) vorzunehmen.

Die erfindungsgemäß behandelten Polyisocyanate weisen vergleichsweise geringe Aktivitätsschwankungen auf. Dies läßt sich besonders einfach anhand verringerter HC-Werte, gleichbedeutend mit verbesserten Aktivitäten, bei vergleichender Prüfung belegen.

Die Erfindung soll anhand der nachfolgenden Beispiele näher erläutert werden. Die angegebenen "HC-Werte" beziehen sich auf den Gehalt an hydrolysierbarem Chlor. Alle Prozentangaben sind Gewichtsprozente.

Beispiele

Ausgangsisocyanate

Polyisocyanat 1

Rohes Polyisocyanatgemisch der Diphenylmethanreihe mit einem NCO-Gehalt von 30,9 %, einer Viskosität von 110 mPa.s bei 25 °C und einem HC-Wert von 0,068 %.

Polyisocyanat 2

Rohes Polyisocyanatgemisch der Diphenylmethanreihe mit einem NCO-Gehalt von 32,2 %, einer Viskosität von 25 mPa.s bei 25 °C und einem HC-Wert von 0,021 %, bestehend aus
59 % 4,4'-Diisocyanatdiphenylmethan
23 % 2,4'-Diisocyanatdiphenylmethan
3 % 2,2'-Diisocyanatdiphenylmethan
15 % höhermolekularen Homologen

Beispiel 1 (erfindungsgemäß)

1.000 g Polyisocyanat 1 werden bei Raumtemperatur mit 20 g eines Epoxidharzes (Glycidether von Bisphenol A: Epoxidzahl = 0,585) und 0,2 g p-Toluolsulfonsäuremethylester (TSE) gemischt und anschließend unter Rühren und Stickstoff 2 Stunden auf 90 °C erwärmt. Nach dem Abkühlen erhält man ein Isocyanatgemisch mit einem NCO-Gehalt von 29,9 %, einem HC-Wert von 0,045 % und einer Viskosität von 145 mPa.s bei 25 °C.

Beispiel 1a (Vergleich)

Das Polyisocyanat 1 wird ohne Zusatz 2 Stunden unter Rühren und Stickstoff auf 90 °C erwärmt. Nach dem Abkühlen erhält man ein Isocyanatgemisch mit einem NCO-Gehalt von 30,9 %, einer Viskosität von 110 mPa.s bei 25 °C und einem HC-Wert von 0,068 %. Der Versuch zeigt, daß durch das Erhitzen keine Veränderung eintritt (vergl. Tab. 1).

Beispiel 1b (Vergleich)

Das Polyisocyanat 1 wird ohne TSE, jedoch mit dem Epoxidharz wie in Beispiel 1 behandelt. Man erhält ein Isocyanatgemisch mit einem NCO-Gehalt von 30,0 %, einem HC-Wert von 0,044 % und einer Viskosität von 150 mPa.s bei 25 °C. Der Versuch zeigt, daß der Zusatz des Epoxidharzes den HC-Wert senkt; das Polyisocyanat ist aber nicht lagerstabil (vergl. Tab. 1).

Beispiel 2 (erfindungsgemäß)

1.000 g Polyisocyanat 1 werden bei Raumtemperatur mit 20 g des Epoxidharzes aus Beispiel 1 und 0,2 g Trifluormethansulfonsäuretrimethylsilylester (TRIF) gemischt und anschließend unter Rühren und Stickstoff 2 Stunden auf 90 °C erwärmt. Nach dem Abkühlen erhält man ein Isocyanatgemisch mit einem NCO-Gehalt von 30,1 %, einem HC-Wert von 0,043 % und einer Viskosität von 140 mPa.s bei 25 °C.

In Tabelle 1 sind die Beispiele 1, 1a, 1b und 2 aufelistet sowie weitere Beispiele 3, 3a, 3b und 4, die entsprechend Beispiel 1 durchgeführt wurden.

Für die Prüfung der Reaktivitätserhöhung wurden die erhaltenen Isocyanate in einer Menge entsprechend 1 mol NCO mit 147 g (1 mol OH) eines auf Trimethylolpropan gestarteten Propylenoxidgruppen enthaltenden Polyethers mit der OH-Zahl von 380 umgesetzt. Dazu wurde der Polyether auf 70 °C vorerwärmt, das aus den Beispielen erhaltene Isocyanat zugegeben und die Zeit bis zur Vernetzung bestimmt.

Tabelle 1 zeigt einen deutlich verringerten HC-Wert und eine erhöhte Reaktivität der Isocyanate aus den erfindungsgemäßen Beispielen 1, 2, 3 und 4. Diese guten Eigenschaften zeigen zwar auch die Vergleichsbeispiele 1b und 3b, jedoch sind diese Isocyanate nicht lagerstabil.

Tabelle 1

| Bsp. | Zusammensetzung | HC-Wert nach 2 Std. 90° C [%] | NCO-Gehalt [%] | | | Viskosität bei 25° C [mPa.s] | | | Vernetzungszeit mit Polyether [sec] |
|---|---|---|---|---|---|---|---|---|---|
| | | | Ausgangswert | nach 2 Std. 90° C | nach 12 Tg. 50° C | Ausgangswert | nach 2 Std. 90° C | nach 12 Tg. 50° C | |
| 1 | 1.000 g Polyisocyanat 1<br>20 g Epoxidharz<br>0,2 g TSE | 0,045 | 30,3 | 29,9 | 29,7 | 115 | 145 | 150 | 446 |
| 1a | Polyisocyanat 1 | 0,068 | 30,9 | 30,9 | 30,7 | 110 | 110 | 115 | 832 |
| 1b | 1.000 g Polyisocyanat 1<br>20 g Epoxidharz | 0,044 | 30,3 | 30,0 | 25,1 | 115 | 150 | 3200 | |
| 2 | 1.000 g Polyisocyanat 1<br>20 g Epoxidharz<br>0,2 g TRIF | 0,043 | 30,3 | 30,1 | 29,9 | 115 | 140 | 145 | 514 |
| 3 | 1.000 g Polyisocyanat 2<br>20 g Epoxidharz<br>0,2 g TSE | 0,015 | 31,6 | 31,3 | 30,9 | 28 | 64 | 72 | 437 |
| 3a | Polyisocyanat 2 | 0,021 | 32,2 | 32,2 | 32,1 | 25 | 25 | 26 | 911 |
| 3b | 1.000 g Polyisocyanat 2<br>20 g Epoxidharz | 0,016 | 31,6 | 31,2 | 28,1 | 29 | 66 | 1050 | |
| 4 | 1.000 g Polyisocyanat 2<br>20 g Epoxidharz<br>0,2 g TRIF | 0,014 | 31,6 | 31,2 | 31,2 | 28 | 63 | 65 | 516 |

## Patentansprüche

**1.** Verfahren zur Standardisierung und Stabilisierung von organischen Polyisocyanaten, dadurch gekennzeichnet, daß man organische Polyisocyanate bei 20 °C bis 150 °C mit

a) 0,01 bis 10 Gew.-%, vorzugsweise 0,1 bis 2 Gew.-% einer mindestens eine Epoxidgruppe aufweisenden organischen Verbindung der Formel (I)

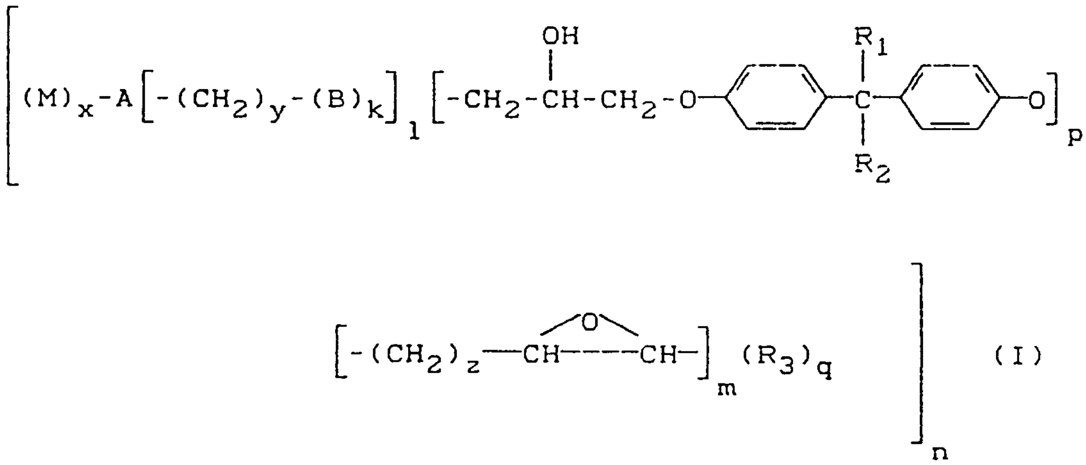

(I)

wobei
x = 0 bis 1
y = 0 bis 4
z = 0 bis 6
k = 0 bis 1
l = 0 bis 8
m = 0,5 bis 4
n = 1 bis 10
p = 0 bis 15
q = 0 bis 1
A = H, ein- oder mehrwertige, gesättigte oder ungesättigte, cyclische oder acyclische Alkyl-, Aryl-, Acyl- oder N-Acylgruppen, gegebenenfalls mit Substituenten,
B = O, S, N
M =

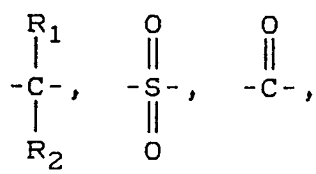

-O-, -S-,
und $R_1$, $R_2$ und $R_3$ für Wasserstoff oder $C_1$- bis $C_4$-Alkylreste stehen,
vermischt und nachfolgend oder gleichzeitig das Umsetzungsprodukt mit
b) 0,01 bis 1 Gew.-%, vorzugsweise 0,01 bis 0,1 Gew.-% einer silylierten Säure der Formel (II)

$X-[Si(CH_3)_3]_n$ (II)

und/oder einer alkylierten Säure der Formel (III)

$X-[Alkyl]_n$ (III)

stabilisiert, wobei
X für einen Säurerest steht, wie er durch Entfernen der aciden Wasserstoffatome aus einer n-basischen Sauerstoff enthaltenden Säure mit einem pKa-Wert von max. 2 erhalten wird, und wobei
n für eine ganze Zahl von 1 bis 3 steht.

2. Verfahren gemäß Anspruch 1, dadurch gekennzeichnet, daß man als organische Polyisocyanate gegebenenfalls chemisch modifizierte Polyisocyanate oder Polyisocyanatgemische der Diphenylmethanreihe verwendet.

3. Verfahren gemäß Anspruch 1, dadurch gekennzeichnet, daß man als Epoxidgruppen aufweisende organische Verbindung Phenoxypropylenoxid oder Bisphenol-A-polyglycidether einsetzt.

4. Verfahren gemäß Anspruch 1, dadurch gekennzeichnet, daß man als Verbindungen der Formel (II) Trifluormethansulfonsäuretrimethylsilylester oder Phosphorsäuretris(trimethylsilylester) verwendet.

5. Verfahren gemäß Anspruch 1, dadurch gekennzeichnet, daß man als Verbindungen der Formel (III) Benzolsulfonsäuremethylester, Methansulfonsäuremethylester, Trifluormethansulfonsäuremethylester oder Toluolsulfonsäuremethylester verwendet.

6. Verfahren gemäß Anspruch 1, dadurch gekennzeichnet, daß man die Verbindungen der Formel (III) durch Reaktion der freien Säuren mit im Reaktionsgemisch vorhandenen Epoxidgruppen enthaltenden Verbindungen in situ bildet.

7. Verfahren nach Anspruch 1, dadurch gekennzeichnet, daß der Stabilisator aus beliebigen Mischungen der Stabilisatoren (II) und (III) besteht.

8. Verwendung der nach dem Verfahren gemäß Ansprüchen 1-7 behandelten Polyisocyanate als Ausgangsmaterialien bei der Herstellung von Polyurethan-Kunststoffen nach dem Isocyanat-Polyadditionsverfahren, zur Herstellung von modifizierten Polyisocyanaten, zur Herstellung von Polyisocyanurat-Kunststoffen und insbesondere zur Herstellung von Polyurethanschaumstoffen.